(19) Europäisches Patentamt
European Patent Office
Office européen des brevets

(11) Publication number: 0 344 707 A2

(12) **EUROPEAN PATENT APPLICATION**

(21) Application number: 89109722.2

(22) Date of filing: 30.05.89

(51) Int. Cl.[4]: C07D 417/14 , C07D 403/12 , C07D 205/08 , C07C 65/21 , C07C 69/92

(30) Priority: 02.06.88 US 201656

(43) Date of publication of application: 06.12.89 Bulletin 89/49

(84) Designated Contracting States: DE FR GB IT

(71) Applicant: E.R. SQUIBB & SONS, INC.
Lawrenceville-Princeton Road
Princeton New Jersey 08543-400(US)

(72) Inventor: Treuner, Uwe, D.
Am Goldberg Sonnenstrasse 6
Etterzhausen(DE)

(74) Representative: Staub, Gabriella, Dr.-Ing. et al
Baaderstrasse 3
D-8000 München 5(DE)

(54) **2-Oxo-1-[[(substituted sulfonyl)-amino]carbonyl]-azetidine derivatives.**

(57) Antibacterial activity is exhibited by 2-azetidinones having a 3-acylamino substituent and having an activating group in the 1-position of the formula

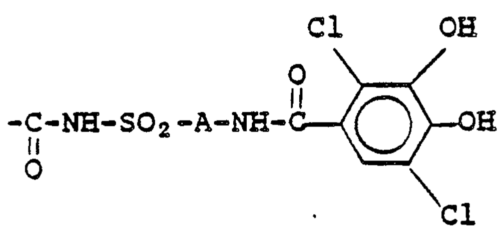

wherein A is -NH-, $-NH-(CH_2)_2-$, 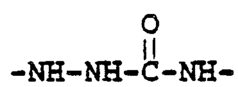,

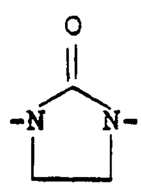, 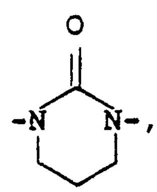, 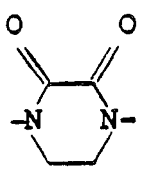 or 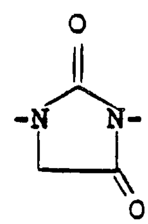.

EP 0 344 707 A2

## 2-OXO-1-[[(SUBSTITUTED SULFONYL)-AMINO]CARBONYL]-AZETIDINE DERIVATIVES

The present invention is directed to new 2-oxo-1-[[(substituted sulfonyl)-amino]carbonyl]-azetidines and particularly concerns such compounds as antibacterial agents.

In accordance with the present invention new compounds having antibacterial activity are disclosed. These new compounds have the general formula

I

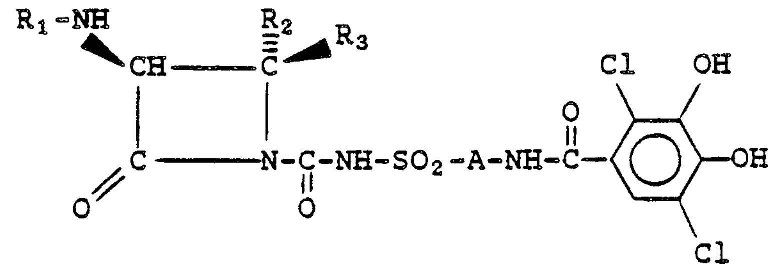

including pharmaceutically acceptable salts thereof, wherein

A is -NH-, $-NH-(CH_2)_2-$,

$-NH-NH-\overset{O}{\overset{\|}{C}}-NH-$,

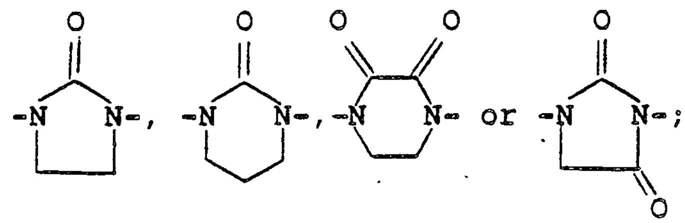

, , or ;

$R_1$ is an acyl group derived from a carboxylic acid;

$R_2$ and $R_3$ are the same or different and each is hydrogen, alkyl, alkenyl, alkynyl, cycloalkyl, phenyl, substituted phenyl or a 4, 5, 6 or 7-membered heterocycle or one of $R_2$ and $R_3$ is hydrogen and the other is azido, halomethyl, dihalomethyl, trihalomethyl, alkoxycarbonyl, 2-phenylethenyl, 2-phenylethynyl, carboxyl,

$$-CH_2X_1,\quad -S-X_2,\quad -O-X_2,\quad -O-\overset{X_3}{\underset{X_5}{C}}-X_4,\quad -S-\overset{X_3}{\underset{X_5}{C}}-X_4 \text{ or}$$

$$-A_1-\overset{O}{\overset{\|}{C}}-NX_6-X_7;$$

$X_1$ is azido, amino, hydroxy, carboxyl, alkoxycarbonyl, alkanoylamino, phenylcarbonylamino, (substituted phenyl)carbonylamino, alkylsulfonyloxy phenylsulfonyloxy, (substituted phenyl)sulfonyloxy, phenyl, substituted phenyl, cyano,

$-A_1-\overset{O}{\overset{\|}{C}}-NX_6X_7$, $-S-X_2$, or $-O-X_2$;

$X_2$ is alkyl, substituted alkyl, phenyl, substituted phenyl, phenylalkyl, (substituted phenyl)alkyl, alkanoyl, substituted alkanoyl, phenylalkanoyl, (substituted phenyl)alkanoyl, phenylcarbonyl, (substituted phenyl)-carbonyl, or heteroarylcarbonyl, and in the case where $X_1$ is $O-X_2$ then $X_2$ can also be alkylideneamino, alkylsulphonylamino, alkoxycarbonyl-alkylsulphonylamino or N,N-cyclodialkanoylamino;

one of $X_3$ and $X_4$ is hydrogen and the other is hyrogen or alkyl, or $X_3$ and $X_4$ when taken together with the carbon atom to which they are attached form a cycloalkyl group;

$X_5$ is formyl, alkanoyl, phenylcarbonyl, (substituted phenyl)carbonyl, phenylalkylcarbonyl, (substituted phenyl)alkylcarbonyl, carboxyl, alkoxycarbonyl, aminocarbonyl, (substituted amino)carbonyl, or cyano;
$X_6$ and $X_7$ are the same or different and each is hydrogen, alkyl, phenyl or substituted phenyl, or $X_6$ is hydrogen and $X_7$ is amino, substituted amino, alkanoylamino or alkoxy, or $X_6$ and $X_7$ when taken together with the nitrogen atom to which they are attached form a 4, 5, 6 or 7-membered heterocycle;
$A_1$ is $-CH=CH-$, $-(CH_2)_m-$, $-(CH_2)_m-O-$, $-(CH_2)_m-NH-$ or $-CH_2-S-CH_2-$;
m is 0, 1 or 2; and,
X is hydrogen, carboxyl or carbamoyl.

The β-lactams of formula I, and pharmaeutically acceptable salts thereof, have activity against gram-positive and gram-negative organisms. These compounds of this invention can be used as agents to combat bacterial infections (including urinary tract infections and respiratory infections) in mammalian species, such as domesticated animals (e.g. dogs, cats, cows, horses, and the like) and humans.

For combating bacterial infections in mammals, a compound of this invention can be administered to a mammal in need thereof in an amount of about 1.4 mg/kg/day to about 350 mg/kg/day, preferably about 14 mg/kg/day to about 100 mg/kg/day. All modes of administration which have been used in the past to deliver penicillins and cephalosporins to the site of the infection are also contemplated for use with β-lactams of this invention. Such methods of administration include oral, intravenous, intramuscular, and as a suppository.

The compounds of this invention form basic salts with various inorganic and organic bases which are also within the scope of this invention. Such salts include ammonium salts, alkali metal salts, alkaline earth metal salts, salts with organic bases, e.g. dicyclohexylamine, benzathine, N-methyl-D-glucamine, hydrabamine and the like. The pharmaceutically acceptable salts are preferred, although other salts are also useful, e.g. in isolating or purifying the product.

Some of the compounds of this invention may be crystallized or recrystallized from solvents containing water. In these cases, water of hydration may be formed. This invention contemplates stoichiometric hydrates as well as compounds containing variable amounts of water that may be produced by processes such as lyophilization.

The β-lactams of formula I contain at least one chiral center--the carbon atom in the 3-position of the β-lactam nucleus to which the acylamino substitutent ("$R_1$-NH-") is attached. This invention is directed to those β-lactams which have been described above, wherein the stereochemistry at the chiral center in the 3-position of the β-lactam nucleus is the same as the configuration at the carbon atom in the 6-position of naturally occurring penicillins (e.g. penicillin G) and as the configuration at the carbon atom in the 7-position of naturally occurring cephalosporins (e.g. cephalosporin C). Also included within the scope of this invention are racemic mixtures which contain the above-described β-lactams.

Listed below are definitions of various terms used to describe the β-lactams of this invention. These definitions apply to the terms as they are used throughout the specification (unless they are otherwise limited in specific instances) either individually or as part of a larger group.

The terms "alkyl" and "alkoxy" refer to both straight and branched chain groups. Those groups having 1 to 10 carbon atoms are preferred.

The terms "cycloalkyl" and "cycloalkenyl" refer to cycloalkyl and cycloalkenyl groups having 3, 4, 5, 6 or 7 carbon atoms.

The term "substituted alkyl" refers to alkyl groups substituted with one or more (preferably 1, 2 or 3) azido, amino ($-NH_2$), halogen, hydroxy, carboxy, cyano, alkoxycarbonyl, aminocarbonyl, alkanoyloxy, alkoxy, phenyloxy, (substituted phenyl)oxy, mercapto, alkylthio, phenylthio, (substituted phenyl)thio, alkylsulfinyl, or alkylsulfonyl groups.

The terms "alkanoyl", "alkenyl", and "alkynyl" refer to both straight and branched chain groups. Those groups having 2 to 10 carbon atoms are preferred.

The term "substituted alkanoyl" refers to alkyl groups substituted with one or more (preferably 1, 2 or 3) azido, amino, halogen, hydroxy, carboxy, cyano, alkoxycarbonyl, aminocarbonyl, alkanoyloxy, alkoxy, phenyloxy, (substituted phenyl)oxy, mercapto, alkylthio, phenylthio, (substituted phenyl)thio, alkylsulfinyl or alkylsulfonyl groups.

The terms "halogen" and "halo" refer to fluorine, chlorine, bromine and iodine.

The term "substituted phenyl" refers to a phenyl group substituted with 1, 2 or 3 amino ($-NH_2$), halogen, hydroxyl, trifluoromethyl, alkyl (of 1 to 4 carbon atoms), alkoxy (of 1 to 4 carbon atoms), alkanoyloxy, aminocarbonyl, or carboxy groups.

The expression "a 4, 5, 6 or 7-membered heterocycle" (referred to as "$R_x$") refers to substituted and unsubstituted, aromatic and non-aromatic groups containing one or more (preferably 1, 2 or 3) nitrogen, oxygen or sulfur atoms. Exemplary substituents are oxo (=O), halogen, hydroxy, nitro, amino, cyano, trifluoromethyl, alkyl of 1 to 4 carbons, alkoxy of 1 to 4 carbons, alkylsulfonyl, phenyl, substituted phenyl, 2-

furfurylideneamino

( 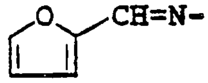 ),

benzylideneamino and substituted alkyl groups (wherein the alkyl group has 1 to 4 carbons). One type of "4, 5, 6 or 7-membered heterocycle" is the "heteroaryl" group. The term "heteroaryl" refers to those 4, 5, 6 or 7-membered heterocycles which are aromatic. Exemplary heteroaryl groups are substituted and unsubstituted pyridinyl, furanyl, pyrrolyl, thienyl, 1,2,3-triazolyl, 1,2,4-triazolyl, imidazolyl, thiazolyl, thiadiazolyl, pyrimidinyl, oxazolyl, triazinyl, and tetrazolyl. Exemplary nonaromatic heterocycles (i.e. fully or partially saturated heterocyclic groups) are substituted and unsubstituted azetidinyl, oxetanyl, thietanyl, piperidinyl, piperazinyl, imidazolidinyl, oxazolidinyl, pyrrolidinyl, tetrahydropyrimidinyl, dihydrothiazolyl and hexahydroazepinyl. Exemplary of the substituted 4, 5, 6 or 7-membered heterocycles are 1-alkyl-3-azetidinyl, 2-oxo-1-imidazolidinyl, 3-alkylsulfonyl-2-oxo-1-imidazolidinyl, 3 benzylideneamino-2-oxo-1-imidazolidinyl, 3-alkyl-2-oxo-1-imidazolidinyl, 3-phenyl (or substituted phenyl)-2-oxo-1-imidazolidinyl, 3-benzyl-2-oxo-1-imidazolidinyl, 3-(2-aminoethyl)-2-oxo-1-imidazolidinyl, 3-amino-2-oxo-1-imidazolidinyl, 3-[(alkoxycarbonyl)-amino]-2-oxo-1-imidazolidinyl, 3-[2-[(alkoxycarbonyl)amino]ethyl]-2-oxo-1-imidazolidinyl, 2-oxo-1-pyrrolidinyl, 2-oxo-3-oxazolidinyl, 4-hydroxy-6-methyl-2-pyrimidinyl, 2-oxo-1-hexahydroazepinyl, 2-oxo-3-pyrrolidinyl, 2-oxo-3-tetrahydrofuranyl, 2,3-dioxo-1-piperazinyl, 2,5 dioxo-1-piperazinyl, 4-alkyl-2,3-dioxo-1-piperazinyl, and 4-phenyl-2,3-dioxo-1-piperazinyl.

The term "substituted amino" refers to a group having the formula $-NX_8X_9$ wherein $X_8$ is hydrogen, alkyl, phenyl, substituted phenyl, phenylalkyl or (substituted phenyl)alkyl, and $X_9$ is alkyl, phenyl, substituted phenyl, phenylalkyl, (substituted phenyl)alkyl, hydroxy, cyano, alkoxy, phenylalkoxy, or amino ($-NH_2$).

The term "acyl" refers to all organic radicals derived from an organic acid (i.e. a carboxylic acid) by removal of the hydroxyl group. Certain acyl groups are, of course, preferred but this preference should not be viewed as a limitation of the scope of this invention. Exemplary acyl groups are those acyl groups which have been used in the past to acylate β-lactam antibiotics including 6-aminopenicillanic acid and derivatives and 7-aminocephalosporanic acid and derivatives; see for example, *Cephalosporins and Pencillins*, edited by Flynn, Academic Press (1972), German Offenlegungsschrift 2,716,677, published October 10, 1978, Belgian patent 867,994, published December 11, 1978, United States patent 4,152,432, issued May 1, 1979, United States patent 3,971,778, issued July 27, 1976, United States patent 4,172,199, issued October 23, 1979, and British patent 1,348,894, published March 27, 1974. The portions of these references describing various acyl groups are incorporated herein by reference. The following list of acyl groups is presented to further exemplify the term "acyl"; it should not be regarded as limiting that term. Exemplary acyl groups are:

(a) Aliphatic groups having the formula

$R_a-\overset{O}{\overset{\parallel}{C}}-$ wherein $R_a$ is alkyl, cycloalkyl, alkoxy, alkenyl, cycloalkenyl, cyclohexadienyl, or alkyl or alkenyl substituted with one or more halogen, cyano, nitro, amino, mercapto, alkylthio, or cyanomethylthio groups;

(b) Carbocyclic aromatic groups having the formula

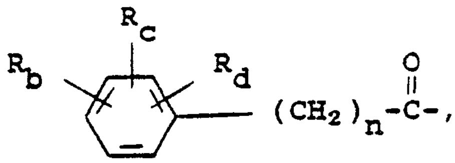

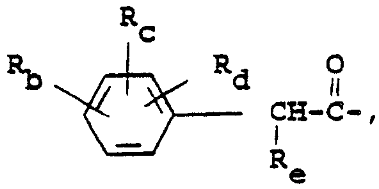

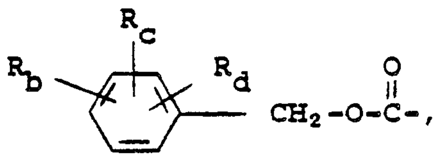

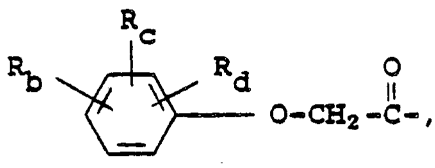

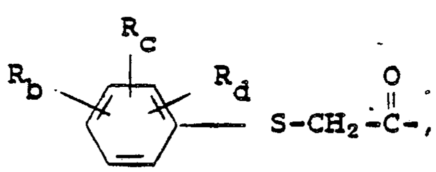

or

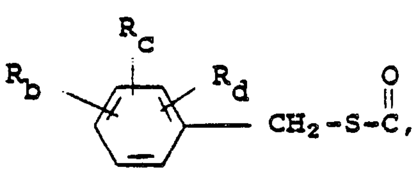

wherein n is 0, 1, 2 or 3; $R_b$, $R_c$ and $R_d$ each is independently hydrogen, halogen, hydroxyl, nitro, amino, cyano, trifluoromethyl, alkyl of 1 to 4 carbon atoms, alkoxy of 1 to 4 carbon atoms or aminomethyl; and $R_e$ is amino, hydroxyl, a carboxyl salt, protected carboxyl, formyloxy, a sulfo salt, a sulfoamino salt, azido, halogen, hydrazino, alkylhydrazino, phenylhydrazino, or [(alkylthio)thioxomethyl]thio;

Preferred carbocyclic aromatic acyl groups include those having the formula

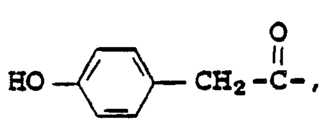

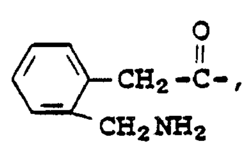

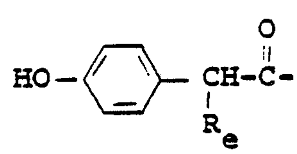

($R_e$ is preferably a carboxyl salt or sulfo salt) and

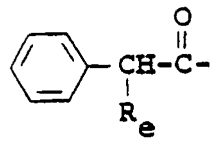

($R_e$ is preferably a carboxyl salt or sulfo salt);

(c) Heteroaromatic groups having the formula

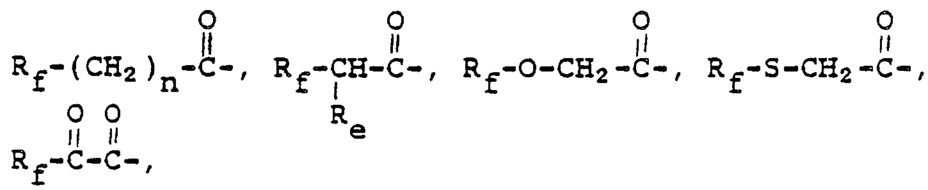

wherein n is 0, 1, 2 or 3; $R_e$ is as defined above; and $R_f$ is a substituted or unsubstituted 5-, 6- or 7-membered heterocyclic ring containing 1, 2, 3 or 4 (preferably 1 or 2) nitrogen, oxygen and sulfur atoms. Exemplary heterocyclic rings are thienyl, furyl, pyrrolyl, pyridinyl, pyrazolyl, pyrazinyl, thiazolyl, pyrimidinyl, thiadiazolyl and tetrazolyl. Exemplary substitutents are halogen, hydroxyl, nitro, amino, protected amino, cyano, trifluoromethyl, alkyl of 1 to 4 carbon atoms, alkoxy of 1 to 4 carbon atoms, or

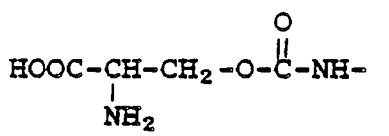 ;

Preferred heteroaromatic acyl groups include those groups of the above formulas wherein $R_f$ is 2-amino-4-thiazolyl, 2-amino-5-halo-4-thiazolyl, 4-aminopyrimidin-2-yl, 5-amino-1,2,4-thiadiazol3-yl, 2-thienyl, 2-furanyl,.or 6-aminopyridin-2-yl;

(d) [[(4-Substituted-2,3-dioxo-1-piperazinyl)carbonyl]amino]arylacetyl groups having the formula

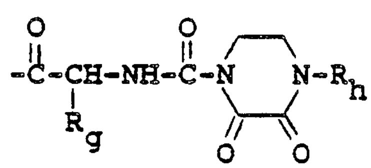

wherein $R_g$ is an aromatic group (including carbocyclic aromatics such as those of the formula

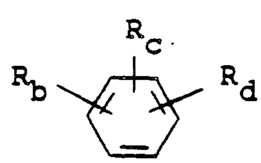

and heteroaromatics as included within the definition of $R_f$); and $R_h$ is alkyl, substituted alkyl (wherein the alkyl group is substituted with one or more halogen, cyano, nitro, amino or mercapto groups), arylmethyleneamino (i.e. -N=CH-$R_g$ wherein $R_g$ is as defined above), arylcarbonylamino (i.e. $-NH-\overset{O}{\overset{\|}{C}}-R_g$ wherein $R_g$ is as defined above) or alkylcarbonylamino;

Preferred [[(4-substituted-2,3-dioxo-1-piperazinyl)carbonyl]amino]arylacetyl groups include those wherein $R_h$ is ethyl, phenylmethyleneamino or 2-furylmethyleneamino;

(e) (Substituted oximino)arylacetyl groups having the formula

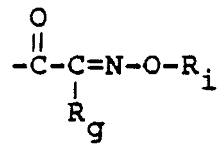

wherein $R_g$ is as defined above and $R_i$ is hydrogen, alkyl, cycloalkyl,

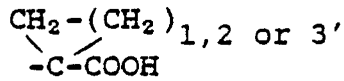

2-pyrrazolylmethyl, (2-oxo-3-pyrrolidinyl)methyl, alkylaminocarbonyl, arylaminocarbonyl (i.e. $-\overset{O}{\overset{\|}{C}}-NH-R_g$ wherein $R_g$ is as defined above) or substituted alkyl (wherein the alkyl group is substituted with one or more halogen, cyano, nitro, amino, mercapto, alkylthio, aromatic group (as defined by $R_g$), carboxyl (including salts thereof), amido, alkoxycarbonyl, phenylmethoxycarbonyl, diphenylmethoxycarbonyl, hydroxyalkoxyphosphinyl, dihydroxyphosphinyl, hydroxy(phenylmethoxy)phosphinyl, dialkoxyphosphinyl or tetrazolyl substituents;

Preferred (substituted oxyimino)arylacetyl groups include those wherein $R_g$ is 2-amino-4-thiazolyl. Also preferred are those groups wherein $R_i$ is methyl, ethyl, carboxymethyl, 1-carboxy-1-methylethyl, 2,2,2-trifluoroethyl or 1-carboxycyclopropyl;

(f) (Acylamino)arylacetyl groups having the formula

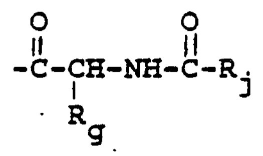

wherein $R_g$ is as defined above and $R_j$ is

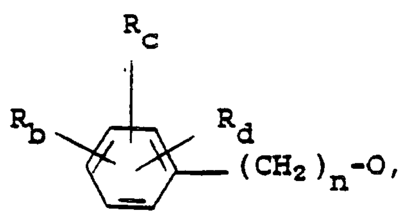

amino, alkylamino, (cyanoalkyl)amino, amido, alkylamido, (cyanoalkyl)amido,

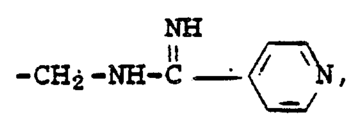

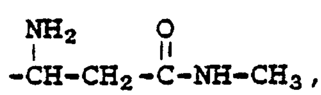

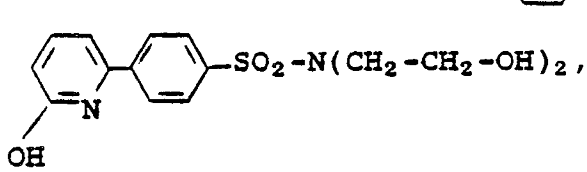

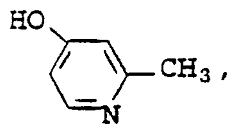 , 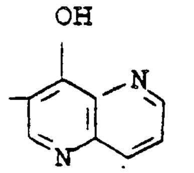 , 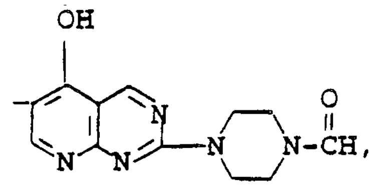 or

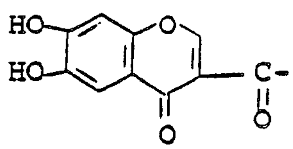 ;

Preferred (acylamino)arylacetyl groups of the above formula include those groups wherein $R_j$ is amino or amido. Also preferred are those groups wherein $R_g$ is phenyl or 2-thienyl;

(g) [[[3-Substituted-2-oxo-1-imidazolidinyl]carbonyl]amino]arylacetyl groups having the formula

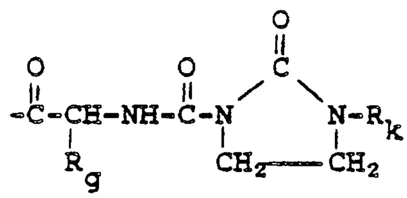

wherein $R_g$ is as defined above and $R_k$ is hydrogen, alkylsulfonyl, arylmethyleneamino (i.e. $-N=CH-R_g$ wherein $R_g$ is as defined above),

$-\overset{O}{\overset{||}{C}}-R_m$ (wherein $R_m$ is hydrogen, alkyl or halogen substituted alkyl), aromatic group (as defined by $R_g$ above), alkyl or substituted alkyl (wherein the alkyl group is substituted with one or more halogen, cyano, nitro, amino or mercapto groups);

Preferred [[3-substituted-2-oxo-1-imidazolidinyl]carbonyl]amino]arylacetyl groups of the above formula include those wherein $R_g$ is phenyl or 2-thienyl. Also preferred are those groups wherein $R_k$ is hydrogen, methylsulfonyl, phenylmethyleneamino or 2-furylmethyleneamino.

The compounds of formula I can be prepared from a protected $\beta$-lactam of the formula

II 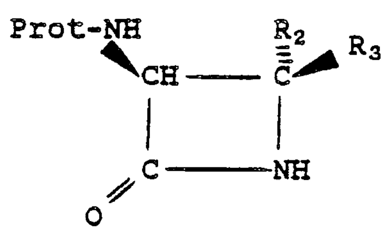 ,

wherein "Prot" refers to an amino-protecting group. These groups are well known in the field of $\beta$-lactam chemistry, and the particular group chosen is not critical. Benzyloxycarbonyl, trityl and t-butoxycarbonyl are exemplary. The reaction of the $\beta$-lactam of formula II with an isocyanate having the formula

III $O=C=N-SO_2-Y$,

wherein Y is a leaving group, such as chlorine, yields a compound having the formula

IV 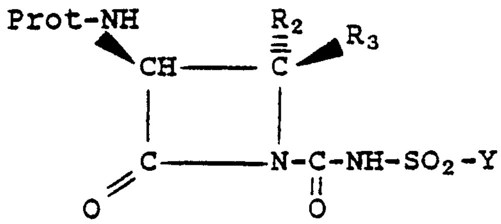 .

The reaction is preferably run in an inert organic solvent, e.g. ethyl acetate, tetrahydrofuran, dimethoxyethane, dichloromethane, acetonitrile or mixtures thereof.

Compound IV can be reacted with an intermediate of the formula

V 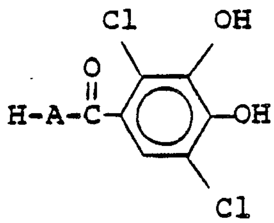

(wherein the hydroxy groups may be protected with acyl, benzyl or silyl) to provide the compounds having the formula

VI 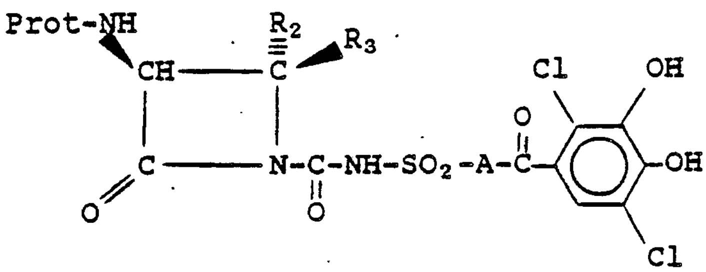 ,

or salts thereof. The reaction above is preferably run in an organic solvent, e.g. methylene chloride, acetonitrile or tetrahydrofuran. If the non-silylated form of compound V is reacted with compound IV, the reaction should also be run in the presence of a strong base, such as triethylamine, trioctylamine or diisopropylethylamine.

Intermediate V, considered to be a part of the present invention, can be prepared by reacting a compound of the formula

VII H-A-$NH_2$ ,

or a half-protected form thereof, i.e.

VII′ H-A-NH-Prot,

with a compound of the formula

VIII 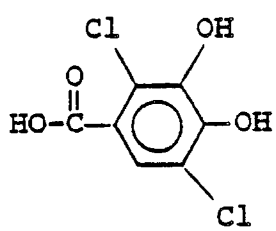

which preferably includes protected forms thereof and may include activated derivatives thereof, such as acid chlorides or active esters. The preparation of compound VIII, i.e. 2,5-dichlorocatechol acid, has been

described in *J. of Antibiotics*, Vol. XL, 1987, 22 by N. Ohi et al.

Novel protected forms of compound VIII, preferred for use in preparing the compounds of the present invention, have the formula

VIIIa

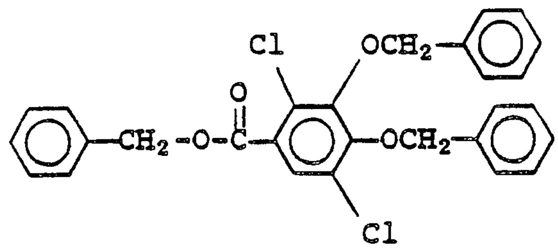

and

VIIIb

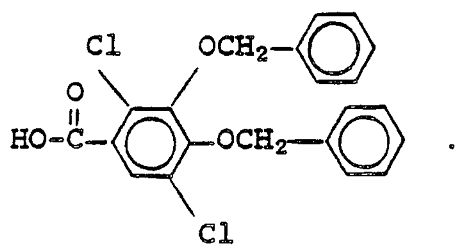

Novel intermediates VIIIa can be prepared by treating a compound of the formula

IX

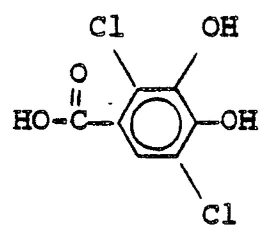

in a solvent, such as dimethylformamide, with a benzylhalide, such as benzylbromide, in the presence of a base, e.g. potassium carbonate, and a catalyst, such as a crownether.

Novel intermediates VIIIb can be prepared by treating a compound of formula VIIIa with dioxane in water and a base, such as potassium hydroxide.

Deprotection of compound VI using conventional techniques yields a compound of the formula

X

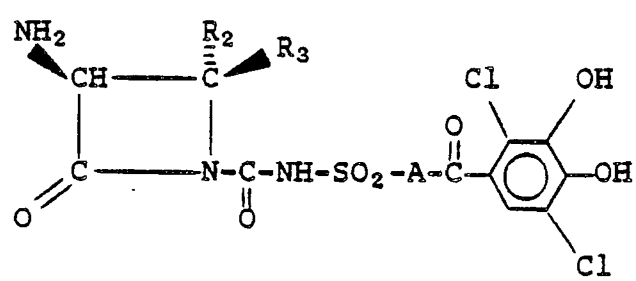

or a salt thereof. If the amino protecting group in compound VI is t-butoxycarbonyl, deprotection can be accomplished with an acid, e.g. formic, trifluoroacetic. Benzyloxycarbonyl protecting groups can be removed by catalytic hydrogenation.

Well known acylation techniques can be used to convert an intermediate of formula X to a corresponding product of formula I. Exemplary techinques include reaction of a compound of formula X with a carboxylic acid ($R_1$-OH), or corresponding carboxylic acid halide or carboxylic acid anhydride. The reaction

with a carboxylic acid proceeds most readily in the presence of a carbodiimide such as dicylcohexylcarbodiimide and a substance capable of forming an active ester *in situ* such as N-hydroxybenzotriazole. In those instances where the acyl group ($R_1$) contains reactive functionality (such as amino or carboxyl groups) it may be necessary to first protect those functional groups, then carry out the acylation reaction, and finally deprotect the resulting product.

Deprotection of the dichlorocatechol portion of compounds of formula I can be accomplished by hydrolysis or fluoride mediated cleavage if the hydroxy protecting group is silyl. Benzyl groups can be removed by hydrogenolysis and acyl groups by hydrolysis.

Alternatively, compounds of formula VI can be prepared by reacting a compound of formula IV in a solvent, such as methylene chloride, tetrahydrofuran, acetonitrile or ethyl acetate, with a compound of formula VII (or a protected derivative thereof, i.e. compound VIII) and thereafter deprotected as described above, to provide an intermediate of the formula

XI 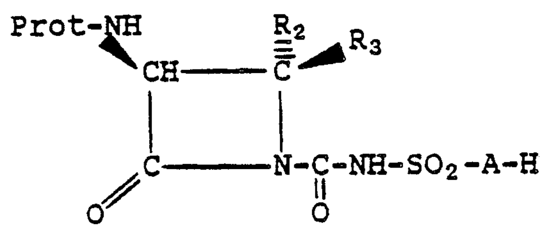 .

Compound XI, including salts and protected derivatives thereof, can thereafter be coupled with a compound of formula VIII, or an activated derivative thereof, in an organic solvent, such as methylene chloride, tetrahydrofuran, dimethylformamide or ethyl acetate, and in the presence of a coupling agent, such as dicyclohexylcarbodiimide or N-hydroxybenzotriazole or a base in the case of activated derivatives, to provide compounds of formula VI. Thereafter, deprotection and acylation can proceed as described above to provide compounds of formula I.

Compounds of formula I may also be formed by partial deprotection of a compound of formula XI (as described above regarding compound VI) and thereafter, acylating as described above to provide intermediates of the formula

XII 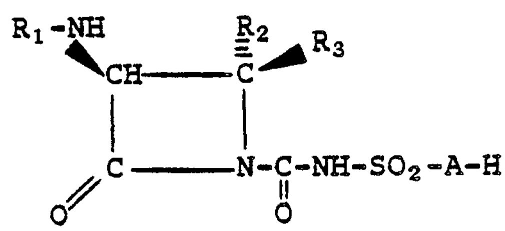 .

Coupling the deprotected forms of compound XII with compound VIII yields the compounds of formula I.

The compounds of formula I wherein

A is 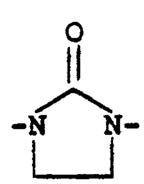, 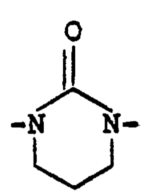 or 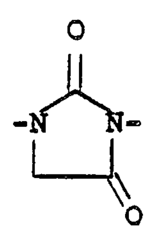; and,

$R_2$ and $R_3$ are each hydrogen or one of $R_2$ and Rs is hydrogen and the other is methyl are preferred. Also preferred are those compounds of formula I wherein $R_1$ is

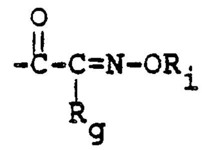

and $R_g$ is 2-amino-4-thiazolyl and $R_i$ is methyl, ethyl, carboxymethyl, 1-carboxy-1-methylethyl, 1-carboxy-1-ethyl or

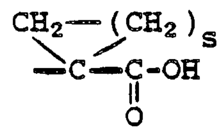

wherein s is 1, 2 or 3. The use of these preferred $R_1$ acyl groups yields a product which exists as the syn or anti isomer or as a mixture of isomers. The syn isomer exhibits greater activity than the anti isomer.

The present invention will now be described by reference to the following example, however, the invention should not be limited to the details therein.

## Example 1

[3S(Z)-2-[[[1-(2-Amino-4-thiazolyl)-2-[[1-[[[[3-[(2,5-dichloro-3,4-dihydroxybenzoyl)amino]-2-oxo-1-imidazolidinyl]sulfonyl]amino]carbonyl]-2-oxo-3-azetidinyl]amino]-2-oxo-ethylidene]amino]oxy]-2-methylpropanoic acid, disodium salt

### A. 1-[(2,2-Dimethylpropylidene)amino]-2-imidazolidinone

A nearly colorless solution of 2-imidazolidinone (252 g, 2.98 mole) and 2N sulfuric acid (4 1) was cooled to 3°C and treated with sodium nitrite (101 g, 1.46 mole) over 15 minutes not allowing the temperature to exceed 5°C. The yellow reaction mixture was stirred at 0-5°C for 1.5 hours after which was added zinc dust (220 g, 3.36 mole) in small portions over 35 minutes keeping the temperature below 5°C. The zinc dissolved completely and rapidly until reaching the equivalence point where the zinc barely dissolved. The resulting grey mixture was stirred at 0°C for 30 minutes, the cold bath was removed and stirring was continued 2 hours longer at room temperature. The mixture was passed through a pad of Celite to remove excess zinc and the clear, colorless filtrate was treated with practical grade pivaldehyde, (152.7 g, ~1.24 moles pivaldehyde). After several minutes of vigorous stirring, a colorless precipitate formed.
Stirring was continued overnight (16 hours). The mixture was filtered to collect the solid and the solid washed with water (3 x 300 ml) and dried on a fluid bed dryer (45 minutes at 40°C) to yield 150 g of the title A compound as a dry freeflowing colorless powder. $R_f$ = 0.48 (silica gel, ethyl acetate:ethanol, 7:3); m.p. 184-185°C.

An analytical sample was prepared by recrystallization from chloroform: m.p. 187.3-187.7°C:

| | | | |
|---|---|---|---|
| Calc'd: | C, 56.78; | H, 8.93; | N, 24.83; |
| Found: | C, 56.54; | H, 8.94; | N, 24.68. |

### B. (2-Oxo-1-imidazolidinyl)carbamic acid, 1,1-dimethylethyl ester

To 2N sulfuric acid (270 ml) at 80°C was added the title A compound (45 g, 0.266 mole) portionwise, over 20 minutes. During addition the pivalaldehyde distilled from the reaction pot as an azeotrope with water. When distillation was complete, the clear, colorless solution was chased with heptane (4 x 20 ml,

azeotrope with water) to chase off any last traces of aldehyde. Total distillation time was I hour 45 minutes. The reaction was cooled to ~30° C and the pH adjusted to 7.25 with the addition of sodium hydroxide.

The aqueous solution was concentrated and the concentrate was chased with absolute ethanol to yield a nearly colorless solid. This material was suspended in methanol (360 ml), warmed to 40° C and then treated with butyloxycarbonyl-anhydride (63.8 g, 1.1 eq).

After stirring 3 hours at 40-45° C, the reaction was completed by chromatographic analysis and was filtered to remove the sodium sulfate. The filtrate was concentrated to a waxy colorless solid. This material was taken into hot acetonitrile (450 ml), seeded and allowed to crystallize at room temperature for 16 hours. It was then chilled at 5° C for 48 hours after which the solid was collected by filtration. The colorless needles were washed once with a small amount of cold acetronitrile, then twice with hexane to afford the first crop of the desired product (42.55 g). The mother liquors were concentrated and the residue taken into a minimal amount of hot acetonitrile to yield a second crop (4.78 g) for a total yield of the title B compound of 47.33 g. $R_f$ = 0.36 (silica gel, ethyl acetate: acetone 1:1); m.p. 186.4-186.5° C:

| | | | |
|---|---|---|---|
| Calc'd: | C, 47.75; | H, 7.51; | N, 20.88; |
| Found: | C, 47.64; | H, 7.47; | N, 20.92. |

C. [1-[[[3-[[(1,1-Dimethylethoxy)carbonyl]amino-2-oxo-1-imidazolidinyl]sulfonyl]amino]carbonyl]-2-oxo-3-azetidinyl]carbamic acid, phenylmethyl ester

A suspension of 2.2 g of (S)-(2-oxo-3-azetidinyl) carbamic acid, phenylmethyl ester compound in 50 ml of ethyl acetate was cooled to -30° C under argon. Chlorosulfonylisocyanate (0.94 ml, 1.53 g) was added and the solution was stirred for about 45 minutes in a -25° C bath. Dichloromethane (20 ml) was added followed by the addition of the title B compound (2.2 g). Triethylamine (2.6 ml, 1.89 g) in 20 ml of methylene chloride was added dropwise to the solution over 8 minutes (rinsed in with 3 ml methylene chloride). The suspension was removed from the cold bath and stirred under argon for 5.5 hours. Another 0.25 ml of triethylamine was added followed by an additional 30 minutes of stirring.

The reaction mixture was poured into 100 ml of water and 36 ml of 0.5 N sulfuric acid was added. The layers were separated, the organic phase washed with 50 ml of water, dried over magnesium sulfate and concentrated *in vacuo* to give 5.22 g of the title C compound.

D. (S)-[1-[[[(3-Amino-2-oxo-1-imidazolidinyl)sulfonyl]amino]carbonyl]-2-oxo-3-azetidinyl]carbamic acid, phenylmethyl ester trifluoroacetic salt

The title C compound (26.1 g) was distilled in methylene chloride (50 ml) and at -5 to 0° C, trifluoroacetic acid (100 ml) was added during a period of 15 minutes. After stirring for 1 hour, the solution was added to isopropanol (600 ml) at 0° C. White crystals of the title D compound were formed and isolated after standing for 15 minutes at -10° C (26.9 g).

E. 3,4-Bis(acetyloxy)-2,5-dichlorobenzoyl chloride

3,4-Diacetoxy-2,5-dichlcrobenzoic acid (2.7 g), thionylchloride (1.4 g) and 1 drop dimethylformamide were dissolved in methylene chloride (50 ml) and refluxed for 10 minutes. After evaporation, the oily residue was treated with petrolether. White crystals of the title E compound (3.21 g) were obtained.

F. (S)-[1-[[[[3-[[3,4-Bis(acetyloxy)-2,5-dichlorobenzoyl]amino]-2-oxo-1-imidazolidinyl]sulfonyl]amino]-carbonyl]-2-oxo-3-azetidinyl]carbamic acid, phenylmethyl ester

The title D compound (7.6 g) and N-methyltrimethylsilyl-trifluoroacetic acid amide (10.5 ml) were stirred in $CH_3CN$ (100 ml) for 15 minutes. A clear solution was obtained. The solvent and the formed trifluoroacetic acid trimethylsilylester were distilled off *in vacuo*, and the residue was dissolved in tetrahydrofuran (50 ml). To this solution was added the title E compound (5.6 g) in methylene chloride (50 ml) dropwise at 0° C.

After stirring overnight, the solvents were distilled off and the residue was stirred with icewater (100 ml) and 3 drops of acetic acid for 1 hour. Isolation by filtration and drying yielded 10.4 g of the title F compound as a white solid.

G. [3S(Z)]-2-[[[1-(2-Amino-4-thiazolyl)-2-[[1-[[[[3-[[3,4-bis(acetyloxy)-2,5-dichlorobenzoyl]amino]-2-oxo-1-imidazolidinyl]sulfonyl]amino]carbonyl]-2-oxo-3-azetidinyl]amino]-2-oxoethylidene]amino]oxy]-2-methylpropanoic acid, diphenylmethyl ester

The title F compound (3.6 g) and N-methyltrimethylsilyl-trifluoroacetic acid amide (2.8 ml) were dissolved in dimethylformamide (75 ml). Palladium on carbon (1.5 g) were added. While stirring continued, a stream of hydrogen was bubbled through the reaction suspension for 1 hour. the catalyst was filtered off and washed with dimethylformamide (20 ml) containing 2 drops of N-methyl-trimethylsilyl-trifluoroacetic acid amide. To the filtrate was added (Z)-2-amino-α-[[2-(diphenylmethoxy)-1,1-dimethyl-2-oxoethoxy]imino]-4-thiazoleacetic acid, 1H-benzotriazol-1-yl ester (2.8 g). This solution was stirred overnight at room temperature. Then the solvent was distilled off *in vacuo*, and the residue stirred with icewater (100 ml) and three drops of acetic acid. A beige solid was obtained after 1 hour of stirring. It was isolated by filtration, washed with water and dried yielding 4.6 g of the title G compound.

H. [3S(Z)-2-[[[1-(2-Amino-4-thiazolyl)-2-[[1-[[[[3-[(2,5-dichloro-3,4-dihydroxybenzoyl)amino]-2-oxo-1-imidazolidinyl]sulfonyl]amino]carbonyl]-2-oxo-3-azetidinyl]amino]-2-oxo-ethylidene]amino]oxo]-2-methylorooanoic acid, disodium salt

The title G compound (4.6 g) was suspended in anisole (9 ml). At -10° C, trifluoroacetic acid amide (60 ml) were dropped into the stirred suspension. After 1 hour, ether (200 ml) was added. After filtration and drying a crude trifluoroacetic acid amide salt of the title compound was obtained. This material was suspended in acetonitrile/water (1:2), and the pH was adjusted to 6.0 with sodium hydrogen carbonate solution. The clear solution was freeze dried after distilling off the organic solvent yielding 4 g of the title compound as a solid. Purification was by chromatography on an XAD-2 resin using water/$CH_3CN$.

**Claims**

1. A compound having the formula

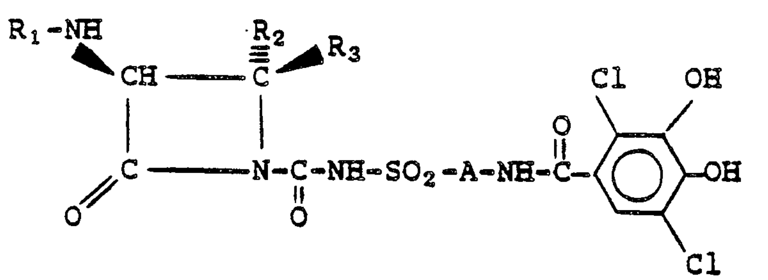

including pharmaceutically acceptable salts thereof, wherein

A is -NH-, $-NH-(CH_2)_2-$, 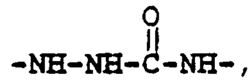,

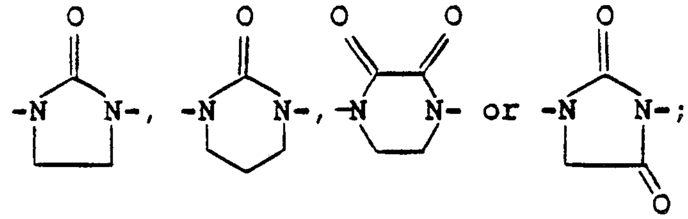 or ;

$R_1$ is an acyl group derived from a carboxylic acid;
$R_2$ and $R_3$ are the same or different and each is hydrogen, alkyl, alkenyl, alkynyl, cycloalkyl, phenyl, substituted phenyl or a 4, 5, 6 or 7-membered heterocycle or one of $R_2$ and $R_3$ is hydrogen and the other is azido, halomethyl, dihalomethyl, trihalomethyl, alkoxycarbonyl, 2-phenylethenyl, 2-phenylethynyl, carboxyl,

$-CH_2X_1$, $-S-X_2$, $-O-X_2$, 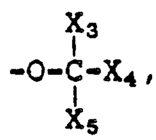, 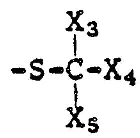 or
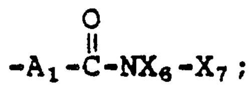;

$X_1$ is azido, amino, hydroxy, carboxyl, alkoxycarbonyl, alkanoylamino, phenylcarbonylamino, (substituted phenyl)carbonylamino, alkylsulfonyloxy, phenylsulfonyloxy, (substituted phenyl)sulfonyloxy, phenyl, substituted phenyl, cyano,
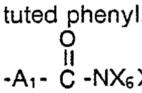, $-S-X_2$, or

$-O-X_2$;
$X_2$ is alkyl, substituted alkyl, phenyl, substituted phenyl, phenylalkyl, (substituted phenyl)alkyl, alkanoyl, substituted alkanoyl, phenylalkanoyl, (substituted phenyl)alkanoyl, phenylcarbonyl, (substituted phenyl)-carbonyl, or heteroarylcarbonyl, and in the case where X′ is $O-X_2$ then $X_2$ can also be alkylideneamino, alkylsulphonylamino, alkoxycarbonyl-alkylsulphonylamino or N,N-cyclodialkanoylamino;
one of $X_3$ and $X_4$ is hydrogen and the other is hyrogen or alkyl, or $X_3$ and $X_4$ when taken together with the carbon atom to which they are attached form a cycloalkyl group;
$X_5$ is formyl, alkanoyl, phenylcarbonyl, (substituted phenyl)carbonyl, phenylalkylcarbonyl, (substituted phenyl)alkylcarbonyl, carboxyl, alkoxycarbonyl, aminocarbonyl, (substituted amino)carbonyl, or cyano;
$X_6$ and $X_7$ are the same or different and each is hydrogen, alkyl, phenyl or substituted phenyl, or $X_6$ is hydrogen and $X_7$ is amino, substituted amino, alkanoylamino or alkoxy, or $X_6$ and $X_7$ when taken together with the nitrogen atom to which they are attached form a 4, 5, 6 or 7-membered heterocycle;
$A_1$ is $-CH=CH-$, $-(CH_2)_m-$, $-(CH_2)_m-O-$, $-(CH_2)_m-NH-$ or $-CH_2-S-CH_2-$;
m is 0, 1 or 2; and,
X is hydrogen, carboxyl or carbamoyl.

2. A compound in accordance with claim 1 wherein

A is -NH-, -NH-$(CH_2)_2$-, 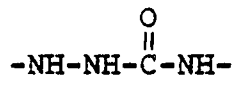,

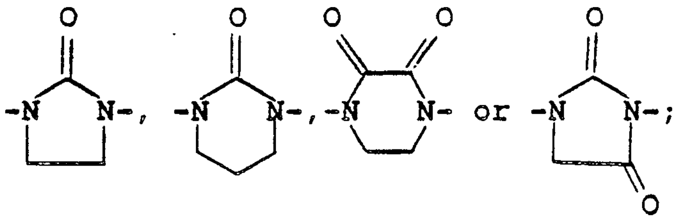

$R_2$ and $R_3$ are each hydrogen or one of $R_2$ and $R_3$ is hydrogen and the other is methyl; $R_1$ is

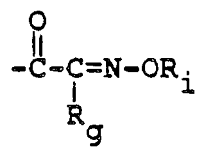

and $R_g$ is 2-amino-4-thiazolyl and $R_i$ is methyl, ethyl, carboxymethyl, 1-carboxy-1-methylethyl, 1-carboxy-1-ethyl or

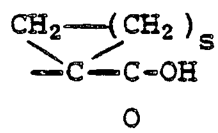

wherein s is 1, 2 or 3.

3. A compound in accordance with claim 1 wherein

A is 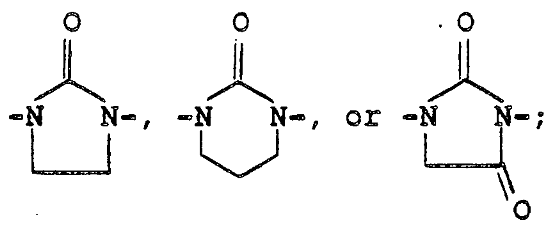

$R_2$ and $R_3$ are each hydrogen or one of $R_2$ and $R_3$ is hydrogen and the other is methyl;
$R_1$ is

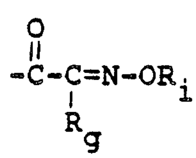

and $R_g$ is 2-amino-4-thiazolyl and $R_i$ is methyl, ethyl, carboxymethyl, 1-carboxy-1-methylethyl, 1-carboxy-1-ethyl or

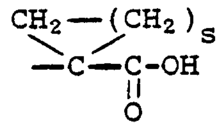

wherein s is 1, 2 or 3. 0

4. A compound of claim 1, being the disodium salt, wherein

$R_1$ is

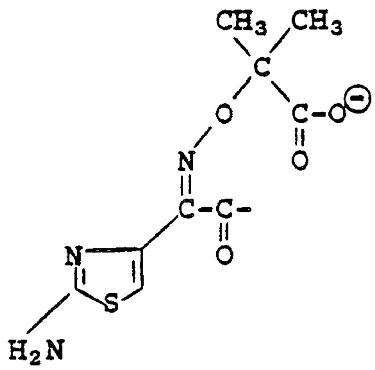

; and,

$R_2$ and $R_3$ are each hydrogen.

5. A compound of claim 1 having the name [3S(Z)-2-[[[1-(2-amino-4-thiazolyl)-2-[[1-[[[[3-[(2,5-dichloro-3,4-dihydroxybenzoyl)amino]-2-oxo-1-imidazolidinyl]-sulfonyl]amino]carbonyl]-2-oxo-3-azetidinyl]amino]-2-oxo-ethylidene]amino]oxy]-2-methylpropanoic acid, disodium salt.

6. A compound of the formula

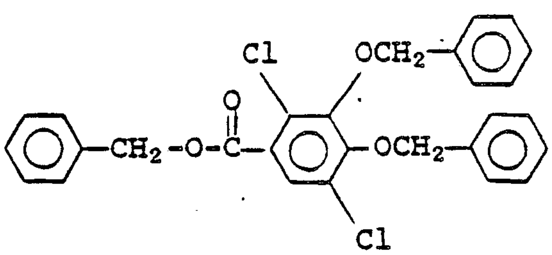

,

that is, 2,5-dichloro-3,4-bis(phenylmethoxy)benzoic acid, phenylmethylester.

7. A compound of the formula

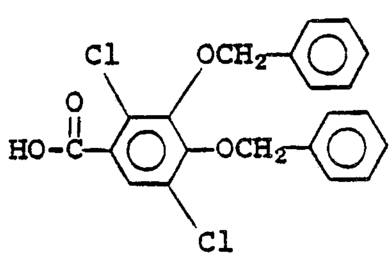

,

that is 2,5-dichloro-3,4-bis(phenylmethoxy)benzoic acid.